# EUROPÄISCHE PATENTSCHRIFT

(11) **EP 0 951 312 B1**
(45) Veröffentlichungstag und Bekanntmachung des Hinweises auf die Patenterteilung: **06.04.2005**
(21) Anmeldenummer: 97945845.2
(22) Anmeldetag: 16.10.1997
(51) Int. Cl.: A61N 1/05, A61N 1/32, A61N 1/36

(54) **OPTISCH ANSTEUERBARE MIKROELEKTRODENANORDNUNG ZUM STIMULIEREN VON ZELLEN, INSBESONDERE RETINA-IMPLANTAT**
OPTICALLY CONTROLLABLE MICROELECTRODE ARRANGEMENT FOR STIMULATING CELLS, IN PARTICULAR A RETINA IMPLANT
SYSTEME DE MICRO-ELECTRODES A COMMANDE OPTIQUE POUR STIMULER DES CELLULES, NOTAMMENT UN IMPLANT RETINIEN

(30) Priorität: 23.10.1996 DE 19644113; 17.02.1997 DE 19705987
(43) Veröffentlichungstag der Anmeldung: 27.10.1999
(73) Patentinhaber: NMI NATURWISSENSCHAFTLICHES UND MEDIZINISCHES INTITUT AN DER UNIVERSITÄT TÜBINGEN, 72770 Reutlingen (DE)
(72) Erfinder: NISCH, Wilfried, D-72072 Tübingen (DE); STELZLE, Martin, D-72770 Reutlingen (DE); WEISS, Stefan, D-72070 Tübingen (DE); ZRENNER, Eberhard, D-72076 Tübingen (DE); GÜNTHER, Elke, D-74762 Reutlingen (DE); STETT, Alfred, D-72768 Reutlingen (DE); GRAF, Heinz, Gerhard, D-71106 Magstadt (DE); GRAF, Michael, D-71332 Waiblingen (DE); SCHUBERT, Markus, B., D-72074 Tübingen (DE); WANKA, Harald, N., D-73630 Remshalden (DE); HIERZENBERGER, Anke, D-71067 Sindelfingen (DE)
(74) Vertreter: Witte, Alexander, Dr.-Ing.
(86) Internationale Anmeldenummer: PCT/EP1997/005701
(87) Internationale Veröffentlichungsnummer: WO 1998/017344

(56) Entgegenhaltungen:
- EP-A- 0 460 320
- WO-A-94/26209
- DE-A- 4 424 753
- US-A- 5 397 350
- WYATT J ET AL: "OCULAR IMPLANTS FOR THE BLIND" IEEE SPECTRUM, Bd. 33, Nr. 5, 1.Mai 1996, Seiten 47, 50-53, 68/69, XP000597263

## Beschreibung

Die Erfindung betrifft eine optisch ansteuerbare Mikroelektrodenanordnung zum Stimulieren von Zellen, insbesondere ein Retina-Implantat mit einer an einem die Zellen enthaltenden Gewebe, z.B. einer Netzhaut, anliegenden Oberfläche, wobei die Oberfläche mit Elektroden zum Stimulieren von Zellen der Gewebes versehen ist und die Elektroden von auf das Gewebe fallendem, sichtbarem Licht derart angesteuert werden, daß ein elektrischer Stimulus von der Elektrode auf die Zelle ausgeübt wird.

Eine Anordnung, nämlich ein Retina-Implantat der vorstehend genannten Art ist aus der EP-A2-0 460 320 bekannt.

Das bekannte Implantat ist ein subretinales Implantat, das demzufolge in untere Schichten der Netzhaut implantiert werden soll. Das bekannte Implantat besteht im wesentlichen aus einem Silizium-Chip, der durch eine große Anzahl von dicht gepackten Mikro-Photodioden gebildet wird. Die photoaktive Oberfläche der Photodioden ist gegen das durch das Auge auftreffende Licht gerichtet. Die Photodioden erzeugen einen amplitudenmodulierten Strom, der die auf der Oberfläche des Implantats aufliegende Zellschicht der Netzhaut stimuliert. Auf diese Weise soll es möglich sein, Patienten, die an unterschiedlichen Formen von Netzhautdegenerationen leiden, wieder ein Sehen oder ein besseres Sehen zu ermöglichen.

In der prioritätsälteren, jedoch nicht-vorveröffentlichten Patentanmeldung 195 29 371 ist eine Mikroelektroden-Anordnung für allgemeine Anwendungen beschrieben. Bei dieser Anordnung sollen biologische Zellen in Netzwerken elektrisch stimuliert werden. Hierzu wird eine Vielzahl von Mikroelektroden eingesetzt. Jede Mikroelektrode umfaßt eine Kontaktierelektrode, die mit dem Netzwerk biologischer Zellen in elektrischen Kontakt bringbar ist, ferner eine Anschlüßelektrode, die elektrisch leitend mit einem Meßgerät oder dgl. verbindbar ist und schließlich ein lichtempfindliches Element, das zwischen der Kontaktierelektrode und der Anschlußelektrode angeordnet ist. Als bevorzugtes Einsatzgebiet derartiger Anordnungen ist das Gebiet der Retina-Implantate angegeben, die bekannte Anordnung ist jedoch auch z.B. zur Stimulation von elektrogenen Zellen "in vivo" geeignet; z.B. bei Herzschrittmachern, Muskelzellstimulatoren, Blasenstimulatoren, wobei z.B. eine optische Ansteuerung mit Licht, insbesondere Infrarot-Licht, durch die Haut erfolgen kann.

Bei dieser bekannten Anordnung werden die einzelnen Mikroelektroden jeweils optisch angesteuert. Dies kann in der eingangs erwähnten Weise dazu dienen, um elektrische Stimuli für Bildpunkte an einer Netzhaut zu erzeugen.

In diesem Zusammenhang ist es gleichgültig, ob das Implantat ein subretinales Implantat ist, das an der Unterseite der Netzhaut implantiert wird oder aber ein epiretinales Implantat, das von der Vorderseite her auf die Netzhaut aufgesetzt wird.

Bei den bekannten Anordnungen werden die Elektroden zwar als "Mikroelektroden" ausgebildet, die erreichbaren Abmessungen der Mikroelektroden sind jedoch durch die jeweils eingesetzten Verfahren begrenzt.

So werden bei den bekannten Anordnungen Mikroelektroden eingesetzt, deren Durchmesser z.B. in der Größenordnung von 10 µm liegt. Sie liegen daher mit ihrer für eine Kontaktgabe an einer Zelle zur Verfügung stehenden Oberfläche in derselben Größenordnung wie die Größenabmessung der Zelle selbst.

Bei einer solchen Konfiguration ist es jedoch in der Regel so, daß die Zelle nicht zentrisch auf der jeweiligen Elektrode sitzt und diese praktisch vollständig abdeckt. Vielmehr ist es regelmäßig so, daß die Zelle nur mit einem Teil ihrer für eine Kontaktgabe zur Verfügung stehenden Oberfläche die entsprechende Kontaktoberfläche der Mikroelektrode überlappt. Dann kommt es jedoch zu Verlusten, weil der "frei" liegende Oberflächenanteil der Mikroelektrode einen Strom in den Elektrolyten der Netzhaut abgibt, der jedoch keinen Stimulus auslöst, sondern vielmehr die Mikroelektrode kurzschließt.

WO-A-94/26209 offenbart eine optisch ansteuerbare Mikroelektrodenanordnung mit den Merkmalen des ersten Teils von Anspruch 1.

Der Erfindung liegt daher die Aufgabe zugrunde, ein Implantat der eingangs genannten Art dahingehend weiterzubilden, daß die zur Verfügung stehende Energie für die Stimuli soweit wie irgend möglich an die Zellen der Netzhaut weitergegeben wird.

Diese Aufgabe wird erfindungsgemäß mit einer Mikroelektroden anordnung nach Anspruch gelöst.

Die zugrundeliegende Aufgabe wird auf diese Weise vollkommen gelöst.

Wenn nämlich, vereinfacht ausgedrückt, die Mikroelektrode wesentlich kleiner ist als die Zelle, ist die Wahrscheinlichkeit eher gering, daß die Mikroelektrode von der Zelle nur teilweise abgedeckt wird. Vielmehr wird es in der Regel so sein, daß mehrere kleine Mikroelektroden von der großen Zelle vollständig überdeckt werden. Dann können diese Elektroden ihren Stimulus nur in vollem Umfange an die Zelle, nicht jedoch an den umgebenden Elektrolyten abgeben.

Bei der bevorzugten Ausgestaltung des erfindungsgemäßen Implantats ist das Verhältnis der Oberflächen von Zelle und Elektrode mindestens 5 : 1, vorzugsweise mehr als 10 : 1.

Diese Maßnahme hat den Vorteil, daß bei praktisch realisierbaren Abmessungen von Elektroden der vorstehend geschilderte Effekt bestmöglich erzielt wird.

Bei einer besonders bevorzugten Ausführungsform der Erfindung sind die Elektroden in dichtestmöglicher Anordnung nebeneinander, jedoch elektrisch voneinander isoliert, auf der Oberfläche angeordnet.

Diese Maßnahme hat den Vorteil, daß die Zellen vollflächig mit Elektroden in Kontakt geraten können. Wenn die Elektroden elektrisch voneinander isoliert sind, hat die von den Elektroden gebildete "Schicht" auf der Oberfläche des Implantats eine gegen Null gehende Querleitfähigkeit.

Bei Ausführungsformen der Erfindung können die Elektroden in an sich bekannter Weise durch Lithographie auf einem Substrat ausgebildet sein.

Bei besonders kleinen Abmessungen der Elektroden kommen jedoch andere Verfahren in Betracht. Insbesondere ist bevorzugt, die Elektroden durch lokale Kristallisation in einer Substratschicht auszubilden. Die Elektroden werden dann z.B. durch Keime der Kristalle gebildet, die sich selbst auf der Oberfläche organisieren.

Ebenso können in sehr dünnen amorphen Schichten (z.B. aus hydrogenisiertem Silizium (a-Si:H) durch kombinierte Anwendung von metallinduzierter Kristallisation und selektiven Ätzverfahren verschiedene Konfigurationen solcher Elektroden hergestellt werden.

Wenn die Substratschicht selbst z.B. eine P-Schicht ist und die Querleitfähigkeit sehr gering eingestellt werden kann, kann die von den Elektroden gebildete Schicht selbst als Teil eines Halbleiterschalters oder einer Mikrophotodiode verwendet werden.

Erfindungsgemäß sind die Electroden Teil eines lichtgesteuerten Schalters mit dem eine Spannung als lokaler Stimulus an eine Zelle anlegbar ist.

Diese Maßnahme hat den Vorteil, daß der Stimulierungsvorgang selbst ausschließlich durch ein optisches Signal ausgelöst werden kann. Dies hat bei Retina-Implantaten besondere Bedeutung, weil das auf die Netzhaut auftreffende Bild helle und dunkle Bereiche umfaßt, mit der Folge, daß an den hellen Stellen durchgeschaltet und stimuliert wird, während an den dunklen Stellen eine Stimulation unterbleibt und die entsprechenden Schalter geschlossen bleiben.

Wie bereits angedeutet wurde, ist bevorzugt, wenn die Schalter durch eine Mehrzahl von Substratschichten und die Elektroden gebildet werden.

Dann ergibt sich der Vorteil, daß die Gesamtanordnung sehr kompakt und dünn ausgebildet werden kann, wenn die von den Elektroden gebildete Schicht selbst eine Schicht des Halbleiterschalters oder der Mikrophotodiode ist.

Wie bereits weiter oben erwähnt wurde, sind Implantate der hier interessierenden Art sowohl als subretinale Implantate als auch als epiretinale Implantate einsetzbar. Im letztgenannten Fall muß lediglich sichergestellt werden, daß das Implantat selbst für sichtbares Licht im wesentlichen durchlässig ist.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: einen äußerst schematisierten Querschnitt durch ein Auge, dessen Netzhaut mit einem erfindungsgemäßen Implantat versehen ist;
- Fig. 2: in stark vergrößertem Maßstab einen seitlichen Schnitt durch einen Teil eines Implantats in subretinaler Anordnung; und
- Fig. 3: eine Darstellung, ähnlich Fig. 2, jedoch für ein Ausführungsbeispiel der Erfindung.

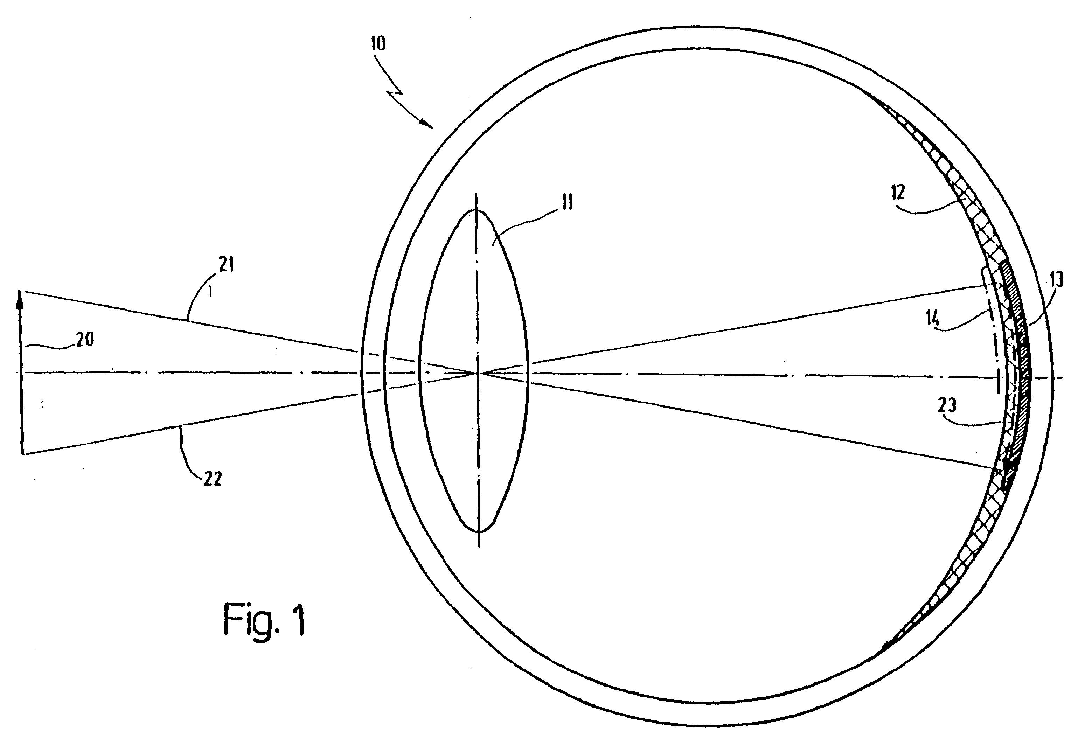

In Fig. 1 bezeichnet 10 insgesamt ein Auge, bspw. ein menschliches Auge. Im Auge 10 sind schematisch eine Linse 11 sowie eine Netzhaut 12 angedeutet.

Bei 13 ist ein subretinales Implantat zu erkennen, das sich in unteren Schichten der Netzhaut 12 befindet. Strichpunktiert ist bei 14 hingegen ein epiretinales Implantat angedeutet, das auf die Oberfläche der Netzhaut aufgesetzt wird.

Ein Gegenstand 20 wird in der üblichen Weise durch die Linse 11 auf der Netzhaut 12 abgebildet, wie mit Strahlen 21, 22 und einem auf dem Kopf stehenden Bild 23 angedeutet.

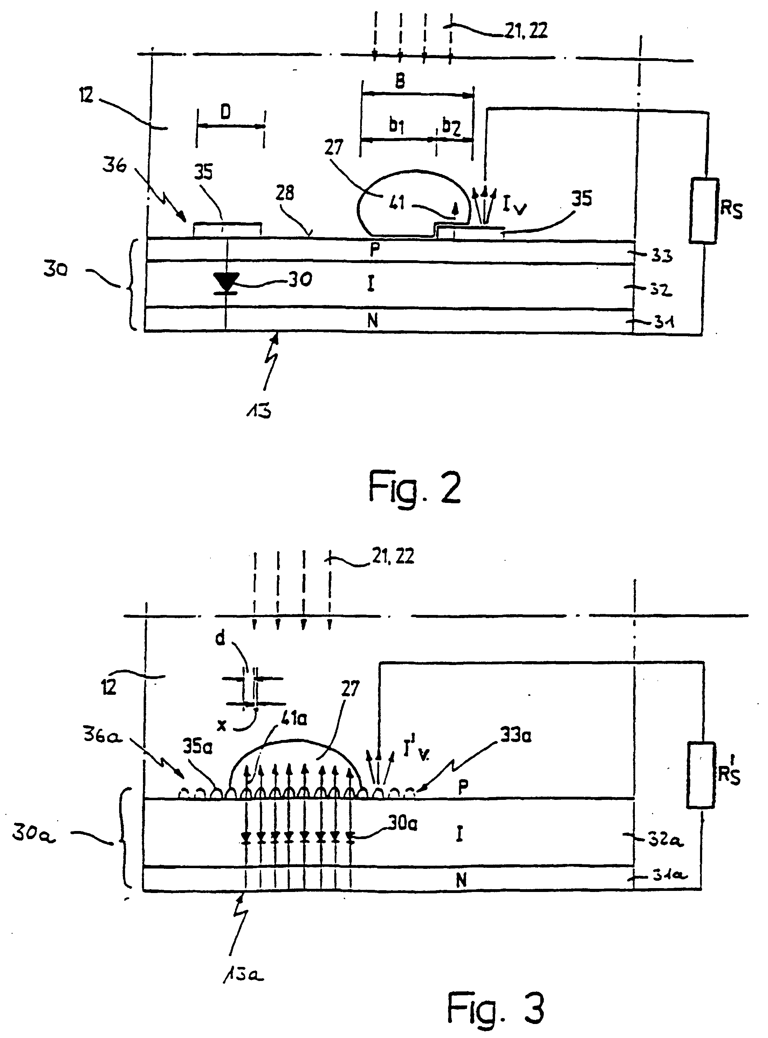

Fig. 2 zeigt in wesentlich vergrößertem Maßstab einen Schnitt durch ein Implantat.

Das Implantat 13 ist in der Darstellung gemäß Fig. 2 subretinal implantiert, es befindet sich also in der Darstellung unterhalb der Netzhaut 12.

In der Netzhaut 12 ist mit 27 eine Zelle angedeutet, die auf einer Oberfläche 28 des Implantats 13 aufliegt und dort kontaktiert werden kann.

Das Implantat 13 selbst hat einen Schichtenaufbau mit einer Diode 30, z.B. bestehend aus einer N-Schicht 31, einer darauf angeordneten I-Schicht 32 sowie einer oben angeordneten P-Schicht 33. Die Diode 30 kann aber auch andere Struktur aufweisen, z.B. (von oben nach unten) N-I-P oder sie kann zusätzliche Schichten aufweisen. Ferner sind P-N- oder N-P-Strukturen aus kristallinem Silizium einsetzbar.

Auf der P-Schicht 33, d.h. auf der Oberfläche 28, sind zwei Elektroden 35 angedeutet. Die Elektroden 35 sind Teil von lichtgesteuerten elektronischen Schaltern, die insgesamt mit 36 angedeutet sind. Sie vermitteln den Kontakt zwischen den Zellen 27 und den Mikrophotodioden 30.

Weitere Einzelheiten zum Aufbau und zur Funktion dieser Schalter 36 sind der bereits weiter oben erwähnten, nicht-vorveröffentlichten deutschen Patentanmeldung 195 29 371 zu entnehmen, auf deren Offenbarung ausdrücklich verwiesen wird.

Wie man aus Fig. 2 erkennt, haben die Elektroden 35 eine Oberfläche, die mit einem Durchmesser D angedeutet ist.

Dieser Durchmesser D liegt in derselben Größenordnung wie die Breite B der Zelle 27, die in der Praxis in der Größenordnung von 10 µm liegt.

Die in Fig. 2 eingezeichnete Zelle 27 überdeckt die Elektrode 35 jedoch nur teilweise, nämlich mit einem Teilbereich b₂, während der größere Teilbereich b₁ der Oberfläche der Zelle 27 lediglich auf der P-Schicht 33 aufliegt, also nicht mit dem Kontakt 35 in Verbindung steht.

Wenn nun, wie mit 21, 22 in Fig. 2 angedeutet, sichtbares Licht auf den optisch betätigbaren Schalter unterhalb der rechts eingezeichneten Elektrode 35 fällt, wird zwar ein Stimulus 41 ausgelöst, dieser Stimulus 41 stellt jedoch nur einen Teil der abgegebenen Energie dar. Ein weiterer Anteil des Photostroms, in Fig. 2 mit Iᵥ als Verlustrom angedeutet, fließt demgegenüber aus der frei liegenden Oberfläche der Elektrode 35 über den Elektrolyten zurück in die gemeinsame oder isolierte Referenzfläche des Implantats 13, wie mit einem Widerstand Rₛ angedeutet.

Um die sich daraus ergebenden Nachteile des Energieverlustes zu vermeiden oder zumindest deutlich zu vermindern, kann die Anordnung, wie in Fig. 3 dargestellt, wie folgt modifiziert werden:

In Fig. 3 sind die Elektroden 35a deutlich kleiner ausgebildet. Der Durchmesser d ist wesentlich kleiner als der Durchmesser D gemäß Fig. 2. Er beträgt z.B. nur ein Fünftel oder ein Zehntel des Durchmessers D. Die Elektroden 35a sind ferner voneinander über einen kleinen Abstand x beabstandet oder durch isolierende Schichten voneinander getrennt, so daß sie elektrisch voneinander isoliert sind. Mit anderen Worten, die durch die Elektroden 35a gebildete Schicht 33a hat eine Querleitfähigkeit, die gegen Null geht. Ebenso hat die Schicht 32a eine verschwindend kleine Querleitfähigkeit.

Jeder der Elektroden 35a entspricht einem entsprechenden Schalter 36a, wie ebenfalls in Fig. 3 eingezeichnet.

Die kleinen Elektroden 35a können entweder durch herkömmliche, lithographische Verfahren realisiert werden. Bei sehr kleinen Abmessungen kann hingegen bevorzugt sein, die Elektroden 35a selbstorganisierend herzustellen, indem man sie als Kristallkeime einer Substratschicht 33a darstellt. Diese Substratschicht 33a kann eine P-Schicht sein, wie in Fig. 3 ebenfalls angedeutet. Dann kann die in Fig. 2 als vollständige Schicht 33 vorgesehene P-Schicht entfallen.

Wie man aus Fig. 3 deutlich erkennt, wird die gesamte Unterseite der Zelle 27 (abgesehen von den geringfügigen Zwischenräumen zwischen den Elektroden 35a) von den Elektroden 35a kontaktiert. Wenn nun von jeder dieser kleinen Elektroden 35a ein Stimulus 41a ausgeht, so ist die Gesamtstimulierung der Zelle 27 deutlich größer als dies bei der Anordnung gemäß Fig. 2 der Fall ist.

Wenn auch bei der Konfiguration gemäß Fig. 3 sichtbares Licht auf Elektroden 35a fällt, die nicht von einer Zelle 27 abgedeckt werden, so entsteht auch hier ein Verluststrom Iᵥ' . Allerdings führt im Gegensatz zur Situation gemäß Fig. 2 das gesamte Licht, das durch die Zelle 27 fällt, zu Stimuli, so daß die Ausbeute insgesamt deutlich verbessert ist.

Das Implantat 13 bzw. 13a kann subretinal oder epiretinal implantiert werden. Im letztgenannten Fall ist es lediglich erforderlich, die Schichten 30 bis 32 oder 33 für sichtbares Licht im wesentlichen durchlässig auszubilden.

## Patentansprüche

1. Optisch ansteuerbare Mikroelektrodenanordnung zum Stimulieren von Zellen (27), mit einer mit einem die Zellen (27) enthaltenden Gewebe in Kontakt gelangenden Oberfläche (28), wobei die Oberfläche (28) mit Elektroden (35a) zum Stimulieren von Zellen (27) des Gewebes versehen ist und die Elektroden (35a) von auf das Gewebe fallendem Licht (21, 22) derart angesteuert werden, dass ein elekt-rischer Stimulus (41a) von der Elektrode (35a) auf die Zelle (27) ausgeübt wird, **dadurch gekennzeichnet, dass** die Oberfläche (28) auf einer lichtempfindlichen Schicht (32a, 33a) ausgebildet ist, die Elektroden (35a) Teil eines Schalters (36a) sind, mit dem eine Spannung als der Stimu-lus (41a) an eine Zelle (27) anlegbar ist, und die Elektroden jeweils wesentlich kleiner sind als eine der Zellen.

2. Anordnung nach Anspruch 1, **dadurch gekennzeichnet, daß** das Verhältnis der Oberflächen (B/d) von Zelle (27) und Elektrode (35a) mindestens 5:1, vorzugsweise mehr als 10:1, ist.

3. Anordnung nach Anspruch 1 oder 2, **dadurch gekennzeichnet, daß** die Elektroden (35a) in dichtestmöglicher Anordnung nebeneinander, jedoch elektrisch voneinander isoliert, auf der Oberfläche (28) angeordnet sind.

4. Anordnung nach Anspruch 3, **dadurch gekennzeichnet, daß** die Elektroden durch Lithographie auf einem Substrat ausgebildet sind.

5. Anordnung nach Anspruch 3, **dadurch gekennzeichnet, daß** die Elektroden (35a) durch lokale Kristallisation in einer Substratschicht (33a) ausgebildet sind.

6. Anordnung nach einem der Ansprüche 1 bis 5, **dadurch gekennzeichnet, dass** die Schalter (36a) durch eine Mehrzahl von Substratschichten (31a, 32a, 33a) und die Elektroden (35a) gebildet werden.

7. Anordnung nach einem oder mehreren der Ansprüche 1 bis 6, **dadurch gekennzeichnet, daß** sie als Retina-Implantat (13; 14) ausgebildet ist.

8. Anordnung nach Anspruch 7, **dadurch gekennzeichnet, dass** das Retina-Implantat als subretinales Implantat (13) ausgebildet ist.

9. Anordnung nach Anspruch 7, **dadurch gekennzeichnet, dass** das Retina-Implantat für sichtbares Licht im wesentlichen durchlässig und als epiretinales Implantat (14) ausgebildet ist.

## Claims

1. Optically controllable microelectrode arrangement for stimulating cells (27), having a surface (28) to be placed in contact with a tissue containing said cells (27), whereby said surface (28) is provided with electrodes (35a) for stimulating cells (27) of said tissue, and whereby said electrodes (35a) are controlled by light (21, 22) impinging on said tissue such that an electrical stimulus (41) is exerted by said electrode (35a) to said cell (27), **characterized in that** said surface (28) is configured on a light sensitive layer (32a, 33a), said electrodes (35a) are part of a switch (36a), whereby via said switch a voltage is appliable to a cell (27) as said stimulus (41), and that said electrodes are each substantially smaller than one of said cells.

2. Arrangement according to claim 1, **characterized in** the ratio (B/d) between the surface area of a cell (27) and an electrode (35a) is at least 5:1, preferably greater than 10:1.

3. Arrangement according to claim 1 or claim 2, **characterized in that** said electrodes (35a) are arranged on said surface (28) in the geometrically densest possible arrangement, however, electrically isolated from each other.

4. Arrangement according to claim 3, **characterized in that** said electrodes are formed by way of a lithography on a substrate.

5. Arrangement according to claim 3, **characterized in that** said electrodes (35a) are formed by local crystallization within a substrate layer (33a).

6. Arrangement according to anyone of claims 1 through 5, **characterized in that** the switches (36a) are configured by a plurality of substrate layers (31a, 32a, 33a) and said electrodes (35a).

7. Arrangement according to anyone or plural of claims 1 through 6, **characterized in that** it is configured as a retina implant (13; 14).

8. Arrangement according to claim 7, **characterized in that** said retina implant is configured as a sub-retinal implant (13).

9. Arrangement according to claim 7, **characterized in that** said retina implant is configured as to be essentially permeable for visible light, and is configured as epiretinal implant (14).

## Revendications

1. Système de microélectrodes à commande optique pour stimuler des cellules (27), avec une surface (28) placée en contact avec un tissu contenant les cellules (27), la surface (28) étant dotée d'électrodes (35a) pour la stimulation de cellules (27) du tissu et les électrodes (35a) étant commandées par la lumière (21, 22) tombant sur le tissu de sorte qu'un stimulus électrique (41a) provenant des électrodes (35a) soit exercé sur les cellules (27), **caractérisé en ce que** la surface (28) est formée sur une couche (32a, 33a) sensible à la lumière, les électrodes (35a) font partie d'un commutateur (36a) avec lequel il est possible d'appliquer une tension servant de stimulus (41a) sur une cellule (27) et les électrodes sont chacune nettement plus petites que l'une des cellules.

2. Système selon la revendication 1, **caractérisé en ce que** le rapport des surfaces (B/d) des cellules (27) et des électrodes (35a) est d'au moins 5:1, de préférence supérieur à 10:1.

3. Système selon la revendication 1 ou 2, **caractérisé en ce que** les électrodes (35a) sont disposées selon un agencement le plus dense possible les unes à côté des autres, tout en étant isolées électriquement les unes des autres, sur la surface (28).

4. Système selon la revendication 3, **caractérisé en ce que** les électrodes sont formées par lithographie sur un substrat.

5. Système selon la revendication 3, **caractérisé en ce que** les électrodes (35a) sont formées par cristallisation locale dans une couche de substrat (33a).

6. Système selon l'une des revendications 1 à 5, **caractérisé en ce que** les commutateurs (36a) sont formés par une pluralité de couches de substrat (31a, 32a, 33a) et par les électrodes (35a).

7. Système selon l'une ou plusieurs des revendications 1 à 6, **caractérisé en ce qu'**il est constitué comme implant rétinien (13 ; 14).

8. Système selon la revendication 7, **caractérisé en ce que** l'implant rétinien est constitué comme un implant sous-rétinien (13).

9. Système selon la revendication 7, **caractérisé en ce que** l'implant rétinien est pour l'essentiel transparent à la lumière visible et est constitué comme implant épirétinien (14).
